(19) Europäisches Patentamt
European Patent Office
Office européen des brevets

(11) **EP 2 666 412 A1**

(12) **EUROPEAN PATENT APPLICATION**

(43) Date of publication:
**27.11.2013 Bulletin 2013/48**

(21) Application number: **13168402.9**

(22) Date of filing: **20.05.2013**

(51) Int Cl.:
*A61B 5/1455* (2006.01)    *A61B 5/00* (2006.01)
*G01N 33/49* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.05.2012 US 201213475988**

(71) Applicant: **Jerusalem College of Technology**
**91160 Jerusalem (IL)**

(72) Inventor: **Nitzan, Meir**
**60631 Beit El (IL)**

(74) Representative: **Modiano, Micaela Nadia et al**
**Modiano & Partners (DE)**
**Thierschstrasse 11**
**80538 München (DE)**

(54) **Pulmonary pulse oximetry method for the measurement of oxygen saturation in the mixed venous blood, and corresponding device**

(57)    A method for obtaining diagnostic information relating to the lungs of a subject includes directing into tissue of the lungs of the subject light of a first wavelength and detecting part of the light that has passed primarily through microcirculatory tissue of the lungs and generating a signal which is a function of intensity of the detected light. The signal is then processed to derive a PPG curve for pulmonary microcirculatory arteries. The method is implemented using various locations for a light source and a detector, including various combinations of positioning on the thoracic wall, insertion into the esophagus. Use of two PPG curves for different wavelengths in the infrared range allows derivation of mixed venous blood oxygen saturation.

Figure 4

EP 2 666 412 A1

**Description**

FIELD AND BACKGROUND OF THE INVENTION

*1.1. Arterial and venous oxygen saturation*

Transfer of oxygen from the lungs to the tissue cells is done mainly via the hemoglobin molecules in the red blood cells and only small part of it is dissolved in arterial plasma. Oxygen saturation ($SO_2$) is the ratio of oxygenated hemoglobin to total hemoglobin ($SO_2=HbO_2/(HbO_2+Hb)$), and its value in the arterial blood, $SaO_2$, is 94-99%. The assessment of $SaO_2$ is mainly important for clinical evaluation of proper respiratory function, since $SaO_2$ depends on the adequacy of the ventilation and respiratory function.

Most of the hemoglobin in venous blood is still oxygenated: normal values of the oxygen saturation in the peripheral venous blood are 70-80%. The value of the oxygen saturation in the venous blood also has physiological and clinical significance, as lower blood flow to the tissue results in higher utilization of the oxygen in the blood and lower value of venous oxygen saturation. The measurement of oxygen saturation in the venous blood of an organ provides therefore information on the adequacy of its blood supply.

The blood from the veins of the whole body is drained into the pulmonary artery after being mixed by the right ventricle. The pulmonary artery blood is named mixed venous blood. The value of the mixed venous oxygen saturation, $SmvO_2$, represents the mean oxygen saturation in the veins of the whole body and is of particular interest. Low values of $SmvO_2$ indicate inadequate oxygen supply to the body, either because of low blood flow or because of improper respiration. If the latter failure is eliminated by using $SaO_2$ measurement, low values of $SmvO_2$ indicate low total blood flow, which is equivalent to low cardiac output. In fact the mixed venous oxygen saturation, $SmvO_2$, is used for the quantitative assessment of the cardiac output by means of the Fick method: the cardiac output is determined from the values of total oxygen consumption, arterial oxygen content and venous oxygen content. The total oxygen consumption can be obtained from oxygen consumption measurements in the inhaled and exhaled air and the values of the arterial and venous oxygen content can be derived from the values of $SaO_2$ and $SmvO_2$ respectively.

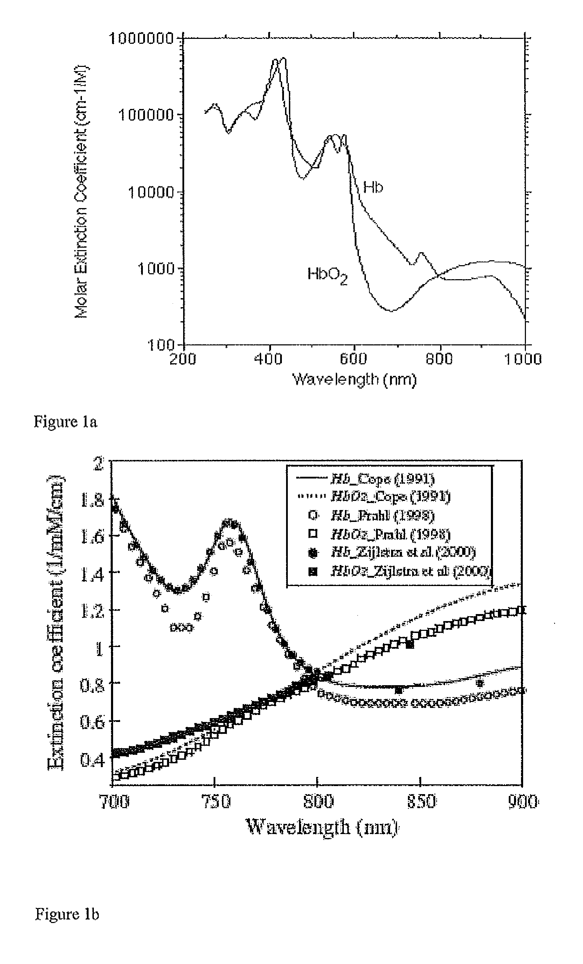

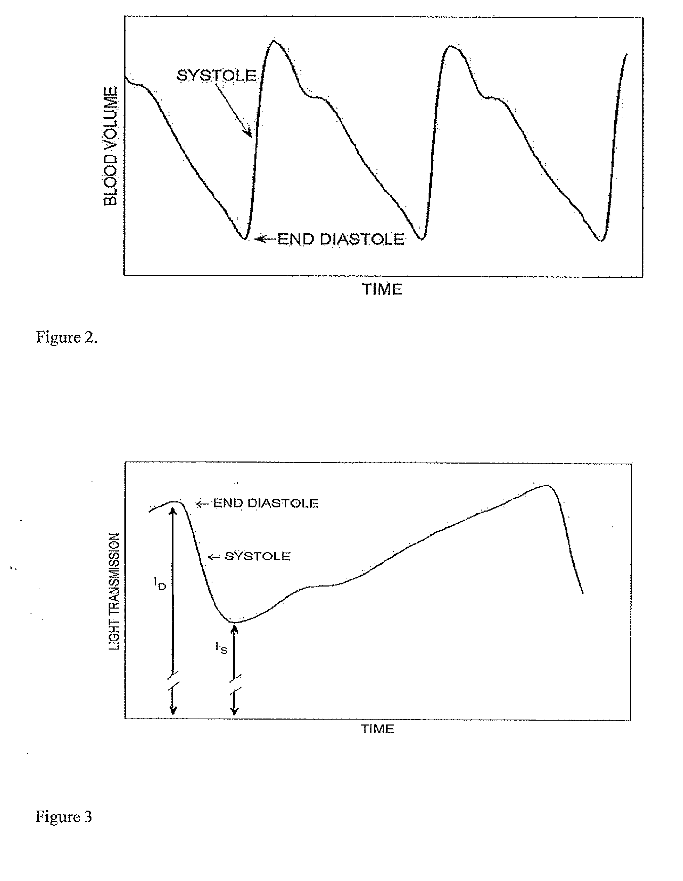

The measurement of $SaO_2$, the oxygen saturation in the arterial blood, can be performed noninvasively by pulse oximetry, which is based on the different light absorption spectrum for oxygenated and de-oxygenated hemoglobin, as shown in Figure 1. Oximetry is a technique in which the attenuation in a substance of light in two wavelengths is measured and the difference in the attenuation of the light between the wavelengths is attributed to the difference in their absorption in the substance. However, in order to quantitatively assess $SaO_2$ the contribution of the arterial blood to the light absorption must be isolated from the contribution of the venous blood. In pulse oximetry the technique for the isolation of the arterial contribution is based on photoplethysmography (PPG) - the measurement of light absorption changes due to the cardiac induced blood volume changes. During systole the blood volume in tissue arteries increases and the transmission through the tissue decreases. Figure 2 shows the inverted PPG signal: during systole there is fast increase in arterial blood volume. Pulse oximetry uses the PPG signal in two wavelengths for the assessment of the oxygen saturation in the arterial blood, $SaO_2$ (Yoshiya, 1980), as will be described later.

In order to assess $SaO_2$ by pulse oximetry there is also a need to consider the effect of scattering on the attenuation of the light. The light traversing a given tissue is not only subject to absorption by the hemoglobin but also to scattering by the various organelles in the tissue cells and in the red blood cells, which diverts the light from its original direct path and also increases its path-length. In order to assess $SaO_2$ or $SvO_2$ by optical means the contribution of the absorption to the attenuation has to be isolated.

While the measurement of $SaO_2$ can be performed noninvasively by pulse oximetry, the available techniques for the measurement of $SmvO_2$, the mixed venous oxygen saturation, are invasive and require the insertion of a catheter into the pulmonary artery (Swan-Ganz catheter). After inserting the catheter $SmvO_2$ can be measured either *in vitro* by extracting mixed venous blood from the pulmonary artery or by *in vivo* measurements, using an optical probe attached to the tip of the catheter. The invasive measurement of $SmvO_2$ is the reason for the invasiveness of the Fick method for the measurement of cardiac output.

*1.2 The PPG signal and its origin*

The PPG probe consists of a light source emitting light into the tissue and a photodetector measuring the light transmitted through the tissue. Two kinds of PPG probes are used for clinical diagnosis and research: transmission and reflection. In transmission PPG, the light source and the detector are attached to two sides of a small organ, such as a finger or an ear lobe, and the detector measures the light transmitted through the organ. In reflection PPG, the light source and the detector are attached to the same side of the organ and the detector measures the scattered light from the tissue under the probe. In both kinds of PPG probe, the detector output oscillates at the heart rate: during systole, blood is ejected from the left ventricle into the peripheral vascular system, thereby increasing the arterial blood content, and consequently decreasing the light intensity transmitted through the tissue. Figure 3 shows the direct PPG signal. The maximal and minimal values of the PPG signal, $I_D$ and Is, respectively, are proportional to the light irradiance transmitted through the tissue when the tissue blood volume is minimal or maximal, respectively. The amplitude of the PPG signal, $(I_D-I_S)$, is related to the maximal change in arterial blood volume during systole, $\Delta V_a$. if $I_D-I_S$ is much smaller than $I_S$, then the relative change in of the PPG signal $(I_D-I_S)/I_S$ is proportional to $\Delta V_a$:

$$(I_D-I_S)/I_S = \alpha_a\Delta V_a \qquad\qquad (1)$$

where $\alpha_a$ is the effective absorption coefficient of the arterial blood, which is a function of the absorption and scattering attenuation constants. $\Delta V_a$, the maximal change in arterial blood volume during systole, is equal to the product of the pulse pressure and the arterial compliance. Hence the amplitude of the PPG signal provides information on the arterial compliance of the systemic circulation (Babchenko et al. 2001, Shelley 2007).

Equation (1) can be applied only for relatively homogenous tissue geometry, as occurs in pure microcirculatory bed. The presence of large blood vessels, where significant light absorption occurs in a single vessel, can limit the validity of Equation (1). Nitzan et al (1998) also showed that the linear relationship between blood volume changes and the PPG pulses holds only for small vessels, in which the absorbed light is small relative to the incident light.

*1.3. Theory of Pulse Oximetry*

Figure 1 presents the extinction coefficient of oxygenated and de-oxygenated hemoglobin as a function of the light wavelength, where the extinction coefficient of a hemoglobin solution is defined as the absorption constant of the hemoglobin solution divided by the concentration of the hemoglobin in the solution.

The theory of deriving arterial oxygen saturation, $SaO_2$, from the PPG curves at two wavelengths is described in several articles (see Wieben, 1997, Mannheimer et al, 1997, Nitzan et al, 2000). The transmitted light intensity, $I_t$, through a tissue sample which includes vessels with whole blood is based on Beer-Lambert law and is given by:

$$I_t = I_0 \exp\left(-\alpha l - \varepsilon c l\right) \qquad\qquad (2)$$

where $l$ is the effective optical path-length (which is higher than the width of the tissue sample because of scattering in the tissue) $\alpha$ is the absorption constant of the tissue, c is the concentration of the blood in the tissue, and $\varepsilon$ is the extinction coefficient of the blood, defined as the absorption constant of the blood divided by the concentration of the blood in the tissue. $I_0$ is the incident light intensity.

During systole the tissue blood volume increases and consequently the light transmission through the tissue decreases, creating the PPG signal/curve. If $I_S$ is the light transmission through the tissue at the maximal increase in tissue blood volume and $I_D$ is the light transmitted through the tissue at end diastole (when the tissue blood volume has its minimal value), then

$$I_S = I_D \exp\left(-\varepsilon_a\Delta c_a l\right) \qquad\qquad (3)$$

$$\ln\left(I_D/I_S\right) = \varepsilon_a\Delta c_a l$$

where $\varepsilon_a$ is the extinction coefficient for the arterial blood, and $\Delta c_a$ is the increase of blood concentration (in the tissue) due to the maximal systolic increase of blood volume. For small blood volume changes $\Delta I_a = I_D - I_S \ll I_S$, and $\ln(I_D/I_S)$ can be approximated by $\Delta I_a/I_S$. If the light transmission is measured for two wavelengths $\lambda_1$ and $\lambda_2$, then

$$(\Delta I_a/I_S)_1 = \varepsilon_{a1}\Delta c_{a1} l_1; \qquad (\Delta I_a/I_S)_2 = \varepsilon_{a2}\Delta c_{a2} l_2 \qquad\qquad (4)$$

and the ratio R, defined by

$$R = \frac{(\Delta I_a / I_s)_1}{(\Delta I_a / I_s)_2} \qquad (5)$$

satisfies the equation

$$R \approx \frac{\varepsilon_{a1} l_1}{\varepsilon_{a2} l_2} \qquad (6a)$$

assuming that the difference in the blood concentration change, $\Delta c_a$, between the two wavelengths can be neglected ($\Delta c_{a1} \approx \Delta c_{a2}$). Note that the last assumption is not always satisfied in reflection PPG, if the penetration depth for the two wavelengths is different. If the two wavelengths are close to each other then the difference of the effective optical path-length is small and

$$R \approx \frac{\varepsilon_{a1}}{\varepsilon_{a2}} \qquad (6b)$$

The relationship between the ratio R - the measured parameter - and the oxygen saturation $SaO_2$ can be derived from the decomposition of the extinction coefficient $\varepsilon$ into its two components, the extinction coefficients for oxygenated blood $\varepsilon_o$ and for deoxygenated blood $\varepsilon_d$:

$$\varepsilon = \varepsilon_o \, SaO_2 + \varepsilon_d \, (1 - SaO_2) = \varepsilon_d + SaO_2 \, (\varepsilon_o - \varepsilon_d) \qquad (7)$$

Then

$$R = \frac{\varepsilon_{d1} + SaO_2(\varepsilon_{o1} - \varepsilon_{d1})}{\varepsilon_{d2} + SaO_2(\varepsilon_{o2} - \varepsilon_{d2})} \qquad (8)$$

and

$$SaO_2 = \frac{\varepsilon_{d1} - R\varepsilon_{d2}}{R(\varepsilon_{o2} - \varepsilon_{d2}) + (\varepsilon_{d1} - \varepsilon_{o1})} \qquad (9)$$

The physiological parameter $SaO_2$ can be derived from the measured parameter R by using Equation 9 and the various values of the extinction coefficient (which are known from the literature) provided that the difference between the path-lengths for the two wavelengths can be neglected. However, commercial pulse oximeters choose one of the wavelengths in the infrared region and the other in the red region (where the difference in the extinction coefficient between oxygenated and deoxygenated blood is maximal) in order to have a higher difference in light transmittance between the two wavelengths. For this choice, the red light scattering constant significantly differs from that of the infrared light resulting in non-negligible difference in optical path-lengths for the two wavelengths.

Due to the difference in scattering between the red and infrared wavelengths, the clinical parameter oxygen saturation $SaO_2$ cannot be derived from the measured parameter R and Equation 9. The actual relationship between the measured parameter R and oxygen saturation $SaO_2$ is achieved for each type of pulse oximeter sensor by calibration (Schowalter, 1997): R is measured in several persons simultaneously with *in vitro* $SaO_2$ measurement in extracted arterial blood by means of co-oximeter which is the gold-standard for $SaO_2$ measurements. For each person R and

$SaO_2$ measurements are taken for several values of $SaO_2$, achieved by changing the partial pressure of oxygen in the inspired air. The table of the simultaneous measurements of R and $SaO_2$ provides the required calibration for the derivation of the clinical parameter oxygen saturation $SaO_2$ from the measured parameter R. The relationship between R and $SaO_2$ can be determined by proposing an analytical formula, such as

$$SaO_2 = \frac{a - bR}{c - dR} \qquad (10)$$

and obtaining the values of the constants a,b,c, and d in the calibration process described above.

The reliability of the calibration is based on the assumption that $I_2/I_1$ does not change between different persons and different physiological and clinical situations. The validity of this assumption is limited and deviations from this assumption are the likely origin of the inherent inaccuracy of the pulse oximetry technique for the assessment of the oxygen saturation, $SaO_2$, in the arterial blood.

If the two wavelengths are close to each other, $I_2/I_1$ is expected to be about 1 and $SaO_2$ can be derived from Equation 9 without calibration. This was shown in our study (Nitzan et al 2000), in which we measured $SpO_2$, using pulse oximetry based on two infrared light emitting diodes (LEDs) (of peak wavelengths 767 and 811 nm) and Equation 9, without calibration. The $SpO_2$ values were somewhat lower than that obtained by commercial calibrated pulse oximeters using red and infrared light, probably because small deviations from the assumption of $I_2/I_1$=1 and because the emission spectrum of the LED is broad, and the extinction coefficient of its light is not accurately definite. The accuracy of the technique increased by using Equation (12) and assessing $(I_2/I_1)$ value from published data (like that of Duncan et al., 1995 for the forearm, $(I_2/I_1)$ = 0.97). The accuracy of the technique is also increased by using two infrared laser diodes of narrow emission spectrum instead of LEDs.

At the end of Section *1.2* it was claimed that the linear relationship between blood volume changes and the PPG pulses holds only for the small blood  vessels of the microcirculation, where the absorbed light in a single vessel is small relative to the incident light. Since pulse oximetry is based on PPG measurements and on the linear relationship between blood volume changes and the PPG signal amplitude, it can be inferred that measurement of oxygen saturation in arterial blood by pulse oximetry can be properly performed only on the microcirculation in tissue. In their articles Mannheimer et al. (2004) and Reuss and Siker (2004) claimed that pulse oximetry is affected by large subcutaneous blood vessels, so that reflectance pulse oximetry should be avoided in sites with palpable arterial pulsatility. While the pulse oximetry is performed on the microcirculatory bed, the calibration, which is required in the conventional pulse oximetry, is done by extracting blood from the big, conduit arteries. The calibration procedure is valid, because the value of oxygen saturation is the same in all arteries, from the big ones to the arterioles, since oxygen dissipation occurs only in the capillaries.

In contrast to the well-established non-invasive measurement of $SaO_2$ by pulse oximetry in the systemic microcirculation , the available techniques for the measurement of $SvO_2$, the mixed venous oxygen saturation, are invasive and require the insertion of a Swan-Ganz catheter into the pulmonary artery. Accordingly, there is a need for an innovative method for the less invasive measurement of the oxygen saturation in mixed venous blood, $SvO_2$.

SUMMARY OF THE INVENTION

The present invention is a method for obtaining physiological information relating to the lungs of a subject, and a corresponding system, as set out in the appended claims.

BRIEF DESCRIPTION OF THE DRAWINGS

The invention is herein described, by way of example only, with reference to the accompanying drawings, wherein:

FIG. 1a is a plot of the extinction coefficients of the oxi- and deoxi-hemoglobin as a function of the wavelength, in the visible and near-infrared regions. The figure was published by Scott Prahl, Oregon Medical Laser Center, 1999, as a best estimate of the spectrum of Hb and $HbO_2$ from a variety of sources. (Appears in M. Nitzan and H. Taitelbaum, 2008. IEEE Instrumentation and Measurement Magazine, 11:9-15.)

FIG. 1b is a plot of the extinction coefficients of the oxi- and deoxi-hemoglobin as a function of the wavelength, in the near-infrared region, published by Kim and Liu, 2007, Phys. Med. Biol. 52:6295-6322.

FIG. 2 is a plot of an inverted PPG signal in the systemic circulation, which presents the light absorption in the tissue against time, showing variations at the heart rate, wherein minimal light absorption occurs at end-diastole when the tissue blood volume is at minimum.

FIG. 3 is a plot of a direct PPG signal in the systemic circulation, which presents the light transmission through the tissue against time, showing variations at the heart rate, wherein maximal intensity of transmitted light occurs at end-diastole when the tissue blood volume is at minimum.

FIG. 4 presents a cross-section of the thorax, with an element for emitting infrared light into the thorax and an element for detecting the light transmitted through the thorax wall and through a portion of the pulmonary tissue.

FIG. 5 presents a cross-section of transmitter and receiver elements, which include optic fiber and reflection prism, for emitting infrared light from a laser diode into the skin and for detecting the light transmitted through the tissue.

FIG. 6a presents a cross-section of the esophagus and a catheter with a PPG probe, which includes a LED and a detector. The PPG probe is applied on a site in the inner esophageal wall, where the lung tissue is in close proximity to the outer esophageal wall. The balloon pressure causes the PPG probe to be in close contact with the inner esophageal wall.

FIG. 6b presents a cross-section of the esophagus and a catheter with a PPG probe, which includes two optic fibers leading from a laser diode and to a detector. The PPG probe is applied on a site in the inner esophageal wall, where the lung tissue is in close proximity to the outer esophageal wall. The balloon pressure causes the PPG probe to be in close contact with the inner esophageal wall.

FIG. 7 is a block diagram schematically showing attachment of a controller including a processing means to the light source and detector of FIGS. 4-6.

## DESCRIPTION OF THE PREFERRED EMBODIMENTS

In the current invention, a method is presented for the measurement of the pulmonary PPG signal, presenting the oscillations at the heart rate of the transmission of light through a region in the microcirculation of the pulmonary system. The pulmonary PPG signal can be utilized for the assessment of the cardiopulmonary circulatory system. It can also be used for the determination of the oxygen saturation in the pulmonary arteries, which is actually the oxygen saturation in mixed venous blood. The latter parameter enables the noninvasive (or minimally invasive) determination of cardiac output by the Fick method. The conventional PPG technique is based on measurements in the arterial system of the systemic circulation conducting blood from the left ventricle to the different organs of the body, except the lungs. The blood volume in the systemic arteries increases by the blood which is ejected from the left ventricle during systole and decreases by the relatively constant blood flow through the capillaries to the veins. The lungs are supplied with venous blood from the right ventricle through the pulmonary artery and, similarly to the systemic circulation, the pulmonary arterial blood volume increases during systole and decreases during diastole. Pulmonary blood volume increase during systole has been demonstrated in several prior studies, using several techniques, including $N_2O$ body plethysmography (Karatzas 1969), analysis of pulmonary arterial pressure curves (Her 1987), ECG-gated radionuclide scintigraphy (Nitzan 1992, 1994), and cardiovascular magnetic resonance (Ugander 2009). One of these techniques (Her 1987) is invasive; the other techniques require either special training of the examinees (Karatzas 1969), or a sophisticated imaging device (Nitzan 1992, 1994, Ugander 2009).

The present invention provides a pulmonary PPG method, in which the pulmonary blood volume increase during systole is detected by the resultant decrease in light transmission through the lungs. The method requires that light emitted from a light-source reaches the lungs and that the light transmitted through the lung tissue is detected. The light transmitted through the lung tissue oscillates at the heart rate, like the systemic PPG, and like the latter the pulmonary PPG is related to the blood volume change in the pulmonary arteries through Equation 1. Like the systemic circulation the systolic blood volume change in the pulmonary arteries is related to the stroke volume and to the arterial compliance and small arteries resistance in the pulmonary system. Hence the pulmonary PPG signal can provide information on these cardiovascular parameters in the cardio-pulmonary system.

The present invention relates generically to any and all techniques in which a pulmonary PPG signal is derived from measurements of light transmission through microcirculatory tissue of the lungs, whether by non-invasive, minimally invasive or fully invasive procedures. While it is simple to achieve the PPG signal in the systemic circulation, because of the proximity of the tissue under examination to the skin, the achievement of the pulmonary PPG is significantly more difficult, since the lungs lay inside the thoracic wall. By way of exemplary but non-limiting preferred examples, the present invention will be exemplified herein with reference to four non-invasive or minimally invasive techniques that enable the measurement of the light transmitted through the pulmonary tissue, each of which is believed to be of value in its own right:

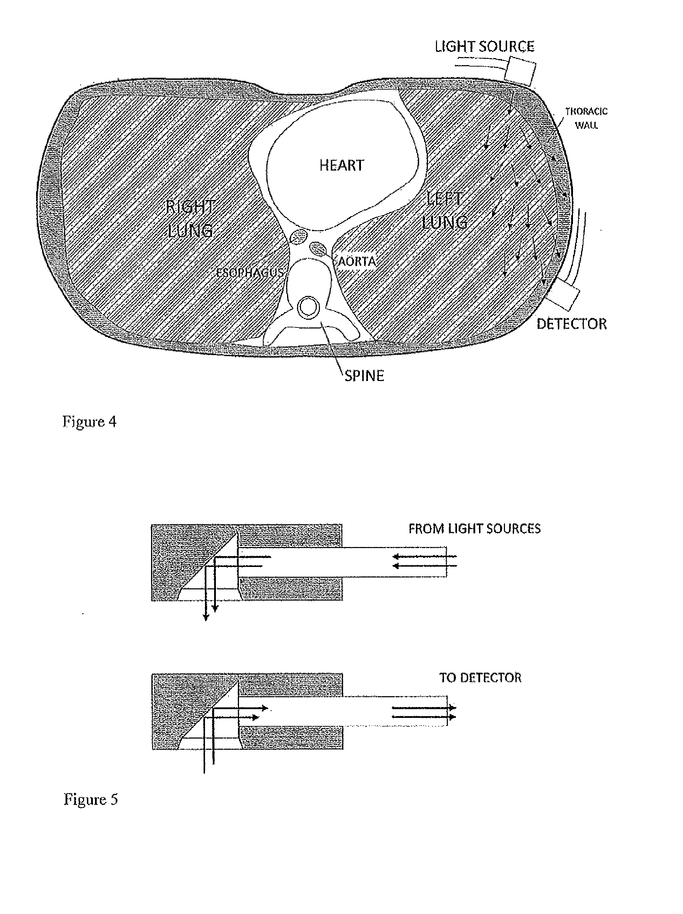

1. A light source and a detector are applied to the thoracic wall of a patient and the detector measures the light which was emitted from the light source and transmitted through the thorax. See Figure 4 and 5. The light source and the detector are separated by at least 20 mm so that the region of illumination overlaps a portion of the pulmonary microcirculation beneath the PPG probe. The technique is especially suitable for infants, whose thoracic wall is relatively thin.

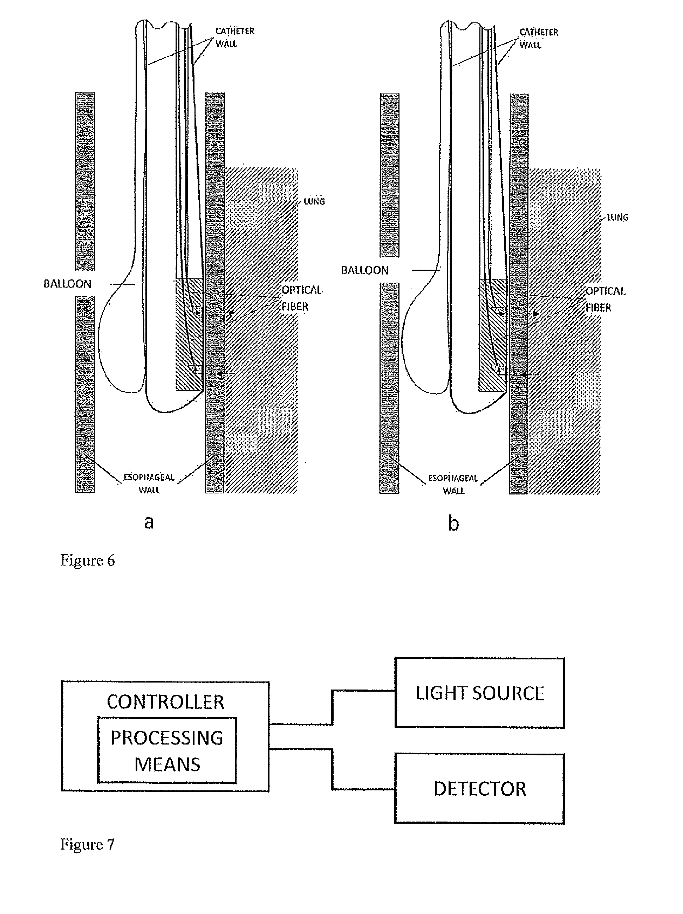

2. A light source and a detector are inserted into the esophagus and brought into close contact with its wall, in a site where the pulmonary tissue is in tight proximity to the esophageal wall, and the detector measures the light which was emitted from the light source and transmitted through the esophageal wall and part of the lungs. See Figure 6. The light source and the detector are separated by at least 10 mm so that the region of illumination overlaps a portion of the pulmonary microcirculation in the neighborhood of the PPG probe.

3. A light source is applied to the thoracic wall of a patient and a detector is inserted into the esophagus and brought into close contact with its wall in a site where the pulmonary tissue is in tight proximity to the esophageal wall. The detector measures the light which was emitted from the light source and transmitted through the thoracic wall and the esophageal wall and through part of the lungs. By suitable choice of the locations of the light-source and the detector the detected light is mainly affected by absorption in the pulmonary microcirculation and the contribution of the attenuation by other organs can be neglected.

4. A catheter with two optic fibers is inserted through the thoracic wall and brought into adjacent relation (i.e., with minimal intervening tissue) with the pulmonary pleura, which covers the lung tissue. The light delivering element and light receiving element in the probe (catheter) are typically brought into contact with the pulmonary pleura or preferably into close proximity to the pulmonary pleura, without contacting it, in order to avoid harm to the vulnerable organ. One of the optic fibers conveys light from a light source into the lung tissue and the other one conveys light which was scattered by the tissue to a detector. Both the light source and the detector are located out of the body.

In the first three above-mentioned pulmonary PPG techniques, referred to herein as "remote PPG techniques", the light, in its way to the pulmonary tissue, also passes through the thoracic wall or the esophageal wall, which are supplied by the systemic circulation. Nevertheless, the main contribution to the PPG signal is by the pulmonary circulation, because the stroke volumes from the right and left ventricles are equal while the former is distributed in the relatively small pulmonary tissue volume and the latter is distributed in the relatively high systemic tissue volume. The relationship between the increase in pulmonary blood volume during systole and the right stroke volume (which is the blood volume ejected from the right ventricle during systole) was determined in several studies: it is 50-67% of the total stroke volume (Karatzas 1969, Nitzan 1992, 1994, Ugander 2009, Her 1987). Hence the systolic blood volume increase in a volume element in the pulmonary circulation is much higher than in a typical volume element in the systemic circulation, enabling the pulmonary PPG measurement

Because of the depth of pulmonary tissue relative to the measurement surface in the remote PPG techniques, the light source and the detector are preferably separated by at least 10 mm in the esophageal probe and by at least 15 mm in the thoracic probe. In order to assess the contribution of the esophageal wall or the thoracic wall circulation to the PPG signal, a second detector can be attached to the esophageal wall or the thoracic wall, where the second detector and the light source are separated by less than 8 mm. In another technique for the assessment of the contribution of the esophageal wall circulation or the thoracic wall circulation to the PPG signal, a second light source is attached to the thoracic wall, where the second light source and the first detector are separated by less than 8 mm. Light transmission measurement by a light-source and a detector of relatively short separation provides information of the tissue of short depth relative to the measurement surface. For a probe adjacent to the pulmonary pleura (option 4, above), smaller spacing between the two optic fibers is preferably used in order to use a single penetrating catheter.

In each of the remote pulmonary PPG techniques, the pathlength of the light is long, and in order to have significant amount of transmitted light intensity for the measurement, infrared light which is less absorbed than visible light, is preferred. Figure 1a presents the extinction coefficients of oxi- and deoxi-hemoglobin. Red light, in the wavelength region of 600-700 nm, is more absorbed by deoxi-hemoglobin, than infrared light, of 700-1000 nm wavelength. For a probe adjacent to the pulmonary pleura (option 4, above), other wavelengths, such as visible wavelengths, may be used.

Parenthetically, it should be noted that the term "light source" is used herein to refer to the light delivering element from which light is released into the tissue. The "light source" thus defined may be a light generating element, such as a laser diode or LED, brought directly to the required location for delivering light, or may be the end of an optic fiber, an applicator connected to such a fiber, or any other waveguide or the like that conveys light to the required site from one or more remotely located light generating device.

Similarly the term "detector" is used herein to refer to the light detecting element which detects the light scattered from the tissue. The "detector" thus defined may be an electro-optic light detecting element, such as a PIN diode or avalanche photodiode, brought directly to the required site of measurement for detecting light, or may be the end of an optic fiber, an applicator connected to such a fiber, or any other waveguide or the like that conveys light to the more remotely electro-optic detecting element from the required site of measurement.

Similar to conventional pulse oximetry technique, which provides information on $SaO_2$ via the measurement of systemic PPG in two wavelengths, the measurement of pulmonary PPG in two wavelengths provides information on the oxygen saturation of the arterial blood in the pulmonary tissue, $SvO_2$, which in fact is equal to the mixed venous blood saturation, $SmvO_2$. $SmvO_2$ provides information on the adequacy of the systemic blood supply and is an essential component in the quantitative determination of cardiac output by the Fick method, as mentioned in Section *1.1*.

The current method for $SmvO_2$ measurement in the pulmonary artery is invasive in the sense that it includes insertion of a Swan-Ganz balloon catheter in the pulmonary artery. $SmvO_2$ is then measured, either intermittently, by extracting blood from the pulmonary artery or continuously, by means of oximetric measurements through optic fibers in the pulmonary artery blood. In another invasive technique, the oxygen saturation in the upper vena cava (central venous oxygen saturation) is measured. The invasive insertion of a catheter in the vena cava is of lower hazard than that through the right ventricle into the pulmonary artery, but the values of oxygen saturation in the two vessels may be different.

Several optical methods have been proposed for the non-invasive or minimally invasive measurement of oxygen saturation of blood within the pulmonary artery or in a central vein. These methods derive the required parameter from spectroscopic absorption measurements utilizing scattered light from the pulmonary artery or the central vein. The proposed various methods try to isolate the contribution of the scattered light from the pulmonary artery (or the other vessel) from that of the surrounding tissue.

Cheng et al in US patent No. US 2006/0253007 presented a method for the assessment of the oxygen saturation in a blood vessel such as the interior jugular vein by illuminating it from the skin. They suggest transmitting of the radiation into two regions, containing different portions of the target structure, for the isolation of the scattered radiation from the target vessel. They also suggest using ultrasound imaging for optimal placement of the optical transmitters and the receivers above the target structure.

Dixon in US Patent No. US2010/0198027 proposed a non-invasive method for the determination of oxygen saturation of blood within a deep vascular structure. Deep vascular structure are major blood vessels which are not superficially located, and include the inferior and superior vena cava, the right atrium, the right ventricle and central and peripheral parts of the pulmonary arteries. The method includes placing emitter and receiver elements of light oximeter device on the skin in the vicinity of the deep vascular structure of interest, wherein placement of the elements is achieved through matching of the plethysmography trace obtained from the oximeter device to known plethysmography characteristics of the deep vascular structure.

Kohl et al in US patent No. US6,961,600B2 presented a minimally-invasive technique for the determination of mixed venous oxygen saturation by introducing catheter with an optical fiber in the bronchia, in the vicinity of the pulmonary artery.

These inventions suggest measuring $SmvO_2$ in the blood within the big arteries or veins, using non-invasive or minimally-invasive techniques, similar to the conventional invasive technique, which measures $SmvO_2$ in the pulmonary artery. However, the measurement of $SmvO_2$ by pulse oximetry, based on light scattering from big vessels is not accurate, as was found in the systemic circulation, that pulse oximetry cannot be used in the vicinity of big blood vessels (Mannheimer 2004, Reuss 2004). Pulse oximetry in the systemic circulation has to be performed on the microcirculatory bed, and the same must be done in the pulmonary system. In preferred implementations of the present invention, the pulmonary pulse oximetry is preferably performed by illuminating the pulmonary tissue, while avoiding scattering of light from the major blood vessels in the thorax, which include the inferior and superior vena cava, the right atrium, the right ventricle and central and peripheral parts of the pulmonary arteries.

Certain embodiments of the present invention perform pulmonary pulse oximetry using pulmonary PPG signals in two wavelengths obtained by a PPG probe applied either on the thoracic wall or on the esophageal wall. As was explained above, two wavelengths in the infrared are preferably used for the measurement of the pulmonary PPG signals and $SvO_2$ due to the long path of the light from the light-source to the detector. This is in contrast to the pulse oximetry in the systemic circulation, which is generally done by red and infrared light, taking the advantage of the relatively high difference between the values of the extinction coefficients of oxi- and deoxi-hemoglobin for red light (see Figure 1). In the pulse oximetry in the systemic circulation the use of red light is possible because the pathlength required for measurements in skin is small, in the order of a few millimeters. Similarly, in the fourth above-mentioned embodiment of the pulmonary pulse oximetry, which uses optic fibers to convey the light to and from the lung, the use of red light is possible. As was explained in Section *1.3*, the pathlengths of the red and infrared light are significantly different, so that calibration by extracted arterial blood is required for the determination of the relationship between $SaO_2$ and the measured parameter R derived from the two PPG signals. Similarly, in the pulmonary pulse oximetry technique, if the difference between the pathlengths of the two wavelengths in the infrared is significant, calibration by extracted blood from the pulmonary artery is required. This calibration is typically not required in pulse oximeter which uses two wavelengths in the infrared region, if they are close enough so that the difference between their pathlengths can be neglected. It is therefore preferable to use two adjacent wavelengths in the infrared, of small difference between their pathlength, and use Equation 9, after neglecting the small difference in their pathlength or correcting it by a suitable correction factor (see Section *1.3*).

In the description of the pulse oximetry method presented above, $SvO_2$ is obtained from R, which was defined as the ratio of the ratios $\Delta I_a/I_S$ for the two wavelengths. $SvO_2$ can also be obtained from the ratio of two values of a parameter related to the change in the PPG signal for the two wavelengths, which can be different than $\Delta I_a/I_S$. It can be chosen as $\ln(I_D/I_S)$ (as in US patent 4,773,422 and 4,167,331) or the derivative of I divided by I (as in US patent 6,505,060).

The esophageal pulmonary pulse oximetry presented in the current patent application differs from the esophageal pulse oximeters presented by Atlee (US patent No. 5,329,922) and Kyriacou et al (Kyriacou 2006), for the measurement of $SaO_2$ in the systemic microcirculation of the esophagus. Accordingly, the distance between the light source and the detector was less than 8 mm enabling the measurement of the PPG signal in the esophageal wall, where the light source and the detector were applied. The two light sources in each wavelength in the esophageal systemic pulse oximeters are required for the increase the PPG signal, while the two detectors in each wavelength in the esophageal pulmonary pulse oximeters are required for the differentiation between the contributions of the systemic and the pulmonary circulations to the PPG signal.

An esophageal systemic pulse oximetry, based on fiber-optic reflectance sensor was presented by Phillips et al (Phillips 2011) and an apparatus for measuring the oxygen saturation level of blood at an internal measurement site, based also on fiber-optic reflectance pulse oximetry was presented by Phillips et al in US patent 2008/0045822. In the former device, the distance between the ends of the detector and the light-sources optic fibers was 4 mm. In the latter apparatus the optical centers of the first and second optical fibers are separated by at least 1 mm at their distal ends. A short distance, of few mms, between the detector and the light-sources or between the ends of the optic fibers of the detector and the light-sources is required for measuring the light absorption in the tissue in contact to the pulse oximeter. In both articles and in the patent application the light-sources included a red emitter, which is highly absorbed in the tissue and can therefore be used only for measurements in tissue of short distance to the pulse oximeter, like the esophageal wall. In the pulmonary pulse oximeter the light-sources emit infrared light and the light sources-detector separation is higher than 10-20 mm, to allow penetration of light to depth of 10-20 mm relative to the measurement surface, which contains pulmonary tissue. It should be noted that the pulse oximeter presented by Phillips et al in their US patent 2008/0045822 was also suggested for the measurement of oxygen saturation in internal tissue like brain, but the probe must be inserted through the skull and applied adjacent to the brain surface in order to measure the oxygen saturation in the brain blood.

The preferred pulse oximeter is a device, which includes two laser diodes of two peak wavelengths in the infrared region and of narrow spectrum and a detector which can detect, for each wavelength, the transmitted light through a portion of the thorax. The light from the laser diodes is conveyed to the thoracic wall by an optic fiber and the light transmitted through the thorax is conveyed to the detector by another optic fiber. For each laser diode a PPG curve is obtained and from each of the two PPG curves the ratio between the PPG pulse amplitude and its baseline is derived. The ratio R of the two values of this amplitude-to-baseline ratio for the two wavelengths is calculated and $SvO_2$ is determined from the equation

$$SvO_2 = \frac{\varepsilon_{d1} - R\varepsilon_{d2}}{R(\varepsilon_{02} - \varepsilon_{d2}) + (\varepsilon_{d1} - \varepsilon_{01})}$$

The value of the extinction coefficients for oxygenated blood $\varepsilon_o$ and for deoxygenated blood $\varepsilon_d$ can be retrieved from the literature data-bases, for each peak wavelength. Another preferred pulse oximeter is an esophageal probe which includes the tips of two optic fibers, one of them conveying infrared light from two laser diodes of two peak wavelengths in the infrared region and of narrow spectrum and the second optic fiber conveying light to a detector. The probe is applied to the esophageal wall, in close proximity to the lung tissue. The two optic fiber tips are separated by more than 10 mm, so that a significant quantity of the light reaching the detector have been scattered by the lung tissue and the PPG pulse will mainly represent the blood volume changes in the pulmonary circulation. For each laser diode a PPG curve is obtained and from each of the two PPG curves the ratio between the PPG pulse amplitude and its baseline is derived. The ratio R of the two values of this amplitude-to-baseline ratio for the two wavelengths is calculated and $SvO_2$ is determined from the equation

$$SvO_2 = \frac{\varepsilon_{d1} - R\varepsilon_{d2}}{R(\varepsilon_{02} - \varepsilon_{d2}) + (\varepsilon_{d1} - \varepsilon_{01})}$$

The value of the extinction coefficients for oxygenated blood $\varepsilon_o$ and for deoxygenated blood $\varepsilon_d$ can be retrieved from the literature data-bases, for each peak wavelength.

In another preferred embodiment $SvO_2$ is obtained from the ratio of two values of a parameter related to the change in light transmission for the two wavelengths, and this parameter can be different than amplitude-to-baseline ratio. It can be chosen as $\ln(I_D/I_S)$ or the derivative of I divided by I.

In another preferred embodiment light emitting diodes (LEDs) are used instead of laser diodes and inserted directly into the esophagus with no need for optic fibers. The emission spectrum of LED is broad, so that the calculation of the mean extinction coefficients over the spectrum band of the emitted light is required.

In another preferred embodiment the LEDs with narrow-band filter are used, so that the calculation of the mean extinction coefficients over the spectrum band of the emitted light is simpler and more accurate.

In another preferred embodiment the pulmonary PPG signal is obtained from several pulmonary PPG pulses, summed together, where the start of each PPG pulse is determined by the corresponding PPG pulse of the systemic circulation.

In another preferred embodiment the pulmonary PPG signal is obtained during specific phase of the respiration.

In another preferred embodiment the pulmonary PPG signal is obtained during the time of minimal movement of the lungs, such as at end expiration or at end inspiration.

In summary, in view of the above, it will be understood that the present invention provides a method and corresponding system for obtaining physiological information relating to the lungs of a subject comprising the steps of: directing into tissue of the lungs of the subject light of a first wavelength; detecting part of the light that has passed primarily through microcirculatory tissue of the lungs and generating a signal which is a function of intensity of the detected light; and processing the signal to derive a PPG curve for pulmonary microcirculatory arteries. The directing and the detecting are preferably performed without breaching any layer of tissue. The light is in the infrared region.

According to a further feature of an embodiment of the present invention, the directing and the detecting are performed using a light source and a detector deployed so as to detect light that has passed primarily through microcirculatory tissue of the lungs primarily avoiding major blood vessels in the thorax.

According to a further feature of an embodiment of the present invention, infrared light of a second wavelength different from the first wavelength is directed into the tissue of the lungs, and the processing is performed so as to derive a PPG curve for pulmonary microcirculatory arteries for each of the first and second wavelengths. According to a further feature of an embodiment of the present invention, the directing and the detecting are performed without breaching any layer of tissue, using light sources and a detector deployed so as to detect light that has passed primarily through microcirculatory tissue of the lungs primarily avoiding major blood vessels.

According to a further feature of an embodiment of the present invention, intensities of the light at the first and second wavelengths are modulated at two different frequencies.

According to a further feature of an embodiment of the present invention, delivery of light at the first and second wavelengths is time-multiplexed.

According to a further feature of an embodiment of the present invention, a ratio R between values of a parameter related to relative changes in light transmission for the first and second wavelengths is derived from the data, and the oxygen saturation in the blood of the pulmonary microcirculatory arteries is determined from the ratio R.

According to a further feature of an embodiment of the present invention, oxygen saturation $SvO_2$ in the blood of the pulmonary microcirculatory arteries is determined from R based on a relationship derived experimentally from invasive measurements of oxygen saturation in the pulmonary artery and R from PPG curves of the pulmonary microcirculatory arteries using the first and second wavelengths.

According to a further feature of an embodiment of the present invention, the relationship is expressed by the formula:

$$SvO_2 = \frac{a - bR}{c - dR}$$

where at least one of the constants a, b, c, and d is obtained experimentally from invasive measurements of oxygen saturation in the pulmonary artery and R from PPG curves of the pulmonary microcirculatory arteries using the first and second wavelengths.

According to a further feature of an embodiment of the present invention, the light of the first wavelength is the peak of an emission spectrum of a first infrared light source and the infrared light of the second wavelength is a peak of an emission spectrum of a second infrared light source, wherein oxygen saturation $SvO_2$ in the blood of the pulmonary microcirculatory arteries is determined from R based on a relationship including the mean value of the extinction coefficient for each of the two emission spectra of the two light sources, for oxygenated blood $\varepsilon_o$ and for deoxygenated blood $\varepsilon_d$.

According to a further feature of an embodiment of the present invention, the relationship between oxygen saturation in the blood of the pulmonary microcirculatory arteries $SvO_2$ and the ratio R which includes the mean value of the extinction coefficient for each of the two wavelength spectra for oxygenated blood $\varepsilon_o$ and for deoxygenated blood $\varepsilon_d$, is

$$SvO_2 = \frac{\varepsilon_{d1} - R\varepsilon_{d2}}{R(\varepsilon_{02} - \varepsilon_{d2}) + (\varepsilon_{d1} - \varepsilon_{01})}$$

According to a further feature of an embodiment of the present invention, the relationship between oxygen saturation in the blood of the pulmonary microcirculatory arteries $SvO_2$ and the ratio R also includes the mean optical path-lengths $l_1$ and $l_2$ for each of the two wavelengths.

According to a further feature of an embodiment of the present invention, the relationship between oxygen saturation in the blood of the pulmonary microcirculatory arteries $SvO_2$ and the ratio R which includes the mean value of the extinction coefficient for each of the two wavelength spectra for oxygenated blood $\varepsilon_o$ and for deoxygenated blood $\varepsilon_d$ and the mean optical path-lengths $l_1$ and $l_2$ for each of the two wavelengths, is

$$SvO_2 = \frac{\varepsilon_{d1} - R\ (l_1/l_2)\varepsilon_{d2}}{R\ (l_1/l_2)(\varepsilon_{02} - \varepsilon_{d2}) + (\varepsilon_{d1} - \varepsilon_{01})}$$

According to a further feature of an embodiment of the present invention, the parameter related to the relative change in light transmission is selected from the group comprising:

$(I_D-I_S)/I_S$, where $I_D$ is the maximal light transmission and $I_S$ is the minimal light transmission;

$[I(t_1)-I(t_2)]/ I(t_2)$, where $I(t_1)$ and $I(t_2)$ are light transmission values at two time points, $t_1$ and $t_2$, along the rise-time of the PPG pulse;

$\ln (I_D/I_S)$;

$\ln [I(t_1)/I(t_2)]$, where $t_1$ and $t_2$ are two time points along the rise-time of the PPG pulse;

$(dI(t)/dt)/I(t)$ at some point along the rise-time of the PPG pulse; and

mean of the values of $(dI(t)/dt)/I(t)$ at some points along the rise-time of the PPG pulse.

According to a further feature of an embodiment of the present invention, the light is directed into the tissue of the lungs by a light source in contact with a first location on the thoracic wall of the subject, and the detecting is performed by a detector in contact with a second location on the thoracic wall of the subject.

According to a further feature of an embodiment of the present invention, the light for each of the first and second wavelengths is directed into the tissue of the lungs by light sources in contact with a first location on the thoracic wall of the subject, and the detecting is performed by a detector in contact with a second location on the thoracic wall of the subject. In this case, the second location is preferably at least 15 millimeters from the first location.

According to a further feature of an embodiment of the present invention, the light for each of the first and second wavelengths is directed into the tissue of the lungs by light sources in contact with a first location on the inner surface of the esophagus of the subject, and the detecting is performed by a detector in contact with a second location on the inner surface of the esophagus of the subject. In this case, the second location is preferably at least 10 millimeters from the first location.

According to a further feature of an embodiment of the present invention, the light is directed into the tissue of the lungs by at least one light source in contact with the thoracic wall of the subject, and the detecting is performed by a detector in contact with the inner surface of the esophagus of the subject.

According to a further feature of an embodiment of the present invention, the directing of the light of the first and second wavelengths and the detecting part of the light are performed using a light delivering element and a light receiving element positioned in adjacent relation to the pulmonary pleura.

According to a further feature of an embodiment of the present invention, the light for each of the first and second wavelengths is directed into the tissue of the lungs by light sources in contact with a first location on an anatomical wall and wherein the detecting is performed by a detector in contact with a second location on the anatomical wall, the first and second locations being spaced apart by a first spacing, and the method further comprises: sampling additional measurements using one of: a second detector in contact with a third location on the anatomical wall spaced from the first location by a distance less than the first spacing, and a second light source in contact with a third location on the anatomical wall spaced from the second location by a distance less than the first spacing, and employing the additional measurements to assess a contribution to the PPG curve due to the blood in the tissue of the anatomical wall.

According to a further feature of an embodiment of the present invention, the signal-to-noise ratio of the shape of the pulmonary PPG curve is increased by adding together several pulmonary PPG signals, where the time-segments of these pulmonary PPG curves are determined by corresponding PPG curves from the systemic circulation, obtained simultaneously with the PPG curves of the pulmonary microcirculatory arteries.

According to a further feature of an embodiment of the present invention, the adding together several pulmonary PPG curves is performed separately for pulmonary PPG curves sampled during different phases of the respiration.

According to a further feature of an embodiment of the present invention, the different phases of the respiration include one of the group: end inspiration, end expiration, end inspiration and end expiration.

According to a further feature of an embodiment of the present invention, the PPG curve for pulmonary microcirculatory arteries is obtained during a specific phase of the respiration.

According to a further feature of an embodiment of the present invention, the specific phase of the respiration includes one of the group: end inspiration, end expiration, end inspiration and end expiration.

**BIBLIOGRAPHY**

***Articles***

1. I. Yoshiya, Y. Shimady and K. Tanake, 1980. Spectrophotometric monitoring of arterial oxygen saturation on the fingertip. Med. Biol. Eng, Comput. 18:27-32.

2. A. Babchenko, E. Davidson, Y. Ginosar, V. Kurtz, I. Feib, D. Adler and M. Nitzan, 2001. Photoplethysmographic measurement of changes in total and pulsatile tissue blood volume following sympathetic blockade. Physiol. Meas. 22:389-396.

3. K. H. Shelley, 2007. Photoplethysmography: beyond the calculation of arterial oxygen saturation and heart rate. Anesth Analg, 105:S31-6.

4. M.N. Nitzan, A. Babchenko, B. Khanokh and D. Landau, 1998. The variability of the photoplethysmographic signal - a potential method for the evaluation of the autonomic nervous system. Physiol. Meas. 19:93-102.

5. O. Wieben. Light absorbance in pulse oximetry. In: Design of Pulse Oximeters. J. G. Webster, editor, 1997. Institute of Physics Publishing, Bristol, pp. 40-55.

6. P. D. Mannheimer, J. R. Casciani, M. E. Fein, S. L. Nierlich, 1997. Wavelength selection for low-saturation pulse oximetry. IEEE Trans. Biomed. Eng. 44(3): 148-158.

7. M. Nitzan, A. Babchenko, B. Khanokh, and H. Taitelbaum, 2000. The measurement of oxygen saturation in venous blood by dynamic near IR spectroscopy. J. Biomed. Optics. 5:155-162.

8. Schowalter JS, Calibration. In: Design of Pulse Oximeters. J. G. Webster, editor, 1997. Institute of Physics Publishing, Bristol, pp. 159-175.

9. A. Duncan , J.H. Meek, M. Clemence, C.E. Elwell. L. Tyszczuk , M. Cope and D.T. Delpy, 1995. Optical pathlength measurements on adult head, calf and forearm and the head of the newborn infant using phase resolved spectroscopy. Phys. Med. Biol. 40:295-304.

10. J.L. Reuss and D. Siker, 2004. The pulse in reflectance pulse oximetry: modeling and experimental studies. J Clin Monit Comput. 18:289-99. 11. P. D. Mannheimer , O' NM, E. Konecny, 2004. The influence of larger subcutaneous blood vessels on pulse oximetry. J Clin Monit Comput. 18:179-88.

12. N.B. Karatzas and G.J. Lee, 1969. Propagation of blood flow pulse in the normal human pulmonary arterial system. Circ, Res. 15:11-21.

13. C. Her, D. Hays and D.E. Lees, 1987. Elevated pulmonary artery systolic storage volume associated with redistribution of pulmonary perfusion. Crit. Care Med. 15:1023-9.

14. M. Nitzan, Y. Mahler, S. Yaffe, R. Marziano, M. Bocher and R. Chishin, 1992. ECG-gated radionuclide plethysmography - a method for the assessment of pulmonary systolic blood volume increase. Clin. Phys. Physiol Meas. 13:21-8.

15. M. Nitzan, Y. Mahler, D. Schechter, S. Yaffe, M. Bocher and R. Chishin, 1994. A measurement of pulmonary blood volume increase during systole in humans. Physiol Meas. 15:489-498

16. M. Ugander, E. Jense and H. Arheden, 2009. Pulmonary intravascular blood volume changes through the cardiac cycle in healthy volunteers studied by cardivascular magnetic resonance measurements of arterial and venous flow. J. Cardiovasc. magnetic resonance, 11:42

17. P.A. Kyriacou, 2006. Pulse oximetry in the esophagus. Physiol Meas. 27:R1-35

18. J.P. Phillips, R.M. Langford, S.H. Chang, P.A. Kyriacou, D.P. Jones, 2011. Photoplethysmographic measurements from the esophagus using a new fiber-optic reflectance sensor. J Biomed Opt. 16(7):077005.

*Patents*

1. B. Dixon. Non-invasive measurement of blood oxygen saturation. US Patent Application Publication No. US 2010/0198027, 2010.

2. X. Cheng et al., Apparatus and method for non-invasive and minimally-invasive sensing of parameters relating to blood. US Patent Application Publication No. US 2006/0253007.

3. B. A. Kohl et al., Transbronchial reflectance oximetric measurement of mixed venous saturation and device therefore.n US patent No. US6,961,600B2, 2005.

4. P. O. Isaacson et al., Single channel pulse oximeter, US patent 4,773,422, 1988

5. L. L. Nielsen, Multi-wavelength incremental absorbence oximeter US patent 4,167,3311979.

6. M. A. Norris, Method and apparatus for determining pulse oximetry differential values US patent 6,505,060, 2003.

7. J.L. Atlee, Oximetric esophageal probe. US patent No. 5,329,922 1934.

8. J.P. Phillips, R.M. Langford, D.P. Jones, and P.A. Kyriacou, 2008. Optical fiber catheter pulse oximeter. US Patent Application Publication No. US 2008/045822.

**Claims**

1.  A method for deriving a PPG curve for pulmonary microcirculatory arteries of a subject, the method comprising the steps of:

    (a) directing into tissue of the lungs of the subject light of a first wavelength;
    (b) detecting part of said light that has passed primarily through microcirculatory tissue of the lungs and generating a signal which is a function of intensity of the detected light; and
    (c) processing said signal to derive a PPG curve for pulmonary microcirculatory arteries.

2.  The method of claim 1, wherein said PPG curve is used for obtaining physiological information relating to the pulmonary circulation of the subject.

3.  The method of claim 1, wherein said first wavelength is a wavelength of infrared light, the method further comprising directing into the tissue of the lungs infrared light of a second wavelength different from said first wavelength, and wherein said processing is performed so as to derive a PPG curve for pulmonary microcirculatory arteries for each of said first and second wavelengths.

4.  The method of claim 3, wherein said directing and said detecting are performed without breaching any layer of tissue, using light sources and a detector deployed so as to detect light that has passed primarily through microcirculatory tissue of the lungs primarily avoiding major blood vessels.

5.  The method of claim 3, further comprising deriving from said PPG curves a ratio R between values of a parameter related to relative changes in light transmission for said first and second wavelengths, and determining from said ratio R the oxygen saturation in the blood of the pulmonary microcirculatory arteries.

6.  The method of claim 5, wherein oxygen saturation $SvO_2$ in the blood of the pulmonary microcirculatory arteries is determined from R based on a relationship derived experimentally from invasive measurements of oxygen saturation in the pulmonary artery blood and R from PPG curves of the pulmonary microcirculatory arteries using said first and second wavelengths.

7.  The method of claim 5, wherein said light of said first wavelength is the peak of an emission spectrum of a first infrared light source and said infrared light of said second wavelength is a peak of an emission spectrum of a second infrared light source, wherein oxygen saturation $SvO_2$ in the blood of the pulmonary microcirculatory arteries is determined from R based on a relationship including the mean value of the extinction coefficient for each of the two emission spectra of the two light sources, for oxygenated blood $\varepsilon_o$ and for deoxygenated blood $\varepsilon_d$.

8. The method of claim 7, wherein the relationship between oxygen saturation in the blood of the pulmonary microcirculatory arteries $SvO_2$ and said ratio R which includes the mean value of the extinction coefficient for each of the two wavelength spectra for oxygenated blood $\varepsilon_o$ and for deoxygenated blood $\varepsilon_d$, is

$$SvO_2 = \frac{\varepsilon_{d1} - R\varepsilon_{d2}}{R(\varepsilon_{o2} - \varepsilon_{d2}) + (\varepsilon_{d1} - \varepsilon_{o1})}$$

9. The method of claim 3, wherein said light for each of said first and second wavelengths is directed into the tissue of the lungs by light sources in contact with a first location on the thoracic wall of the subject, and said detecting is performed by a detector in contact with a second location on the thoracic wall of the subject, said second location being at least 15 millimeters from said first location.

10. The method of claim 3, wherein said light for each of said first and second wavelengths is directed into the tissue of the lungs by light sources in contact with a first location on the inner surface of the esophagus of the subject, and said detecting is performed by a detector in contact with a second location on the inner surface of the esophagus of the subject, said second location being at least 10 millimeters from said first location.

11. The method of claim 3, wherein said light is directed into the tissue of the lungs by at least one light source in contact with the thoracic wall of the subject, and said detecting is performed by a detector in contact with the inner surface of the esophagus of the subject.

12. The method of claim 3, wherein said light for each of said first and second wavelengths is directed into the tissue of the lungs by light sources in contact with a first location on an anatomical wall and wherein said detecting is performed by a detector in contact with a second location on the anatomical wall, said first and second locations being spaced apart by a first spacing, the method further comprising:

    (a) sampling additional measurements using one of:

        (i) a second detector in contact with a third location on the anatomical wall spaced from said first location by a distance less than said first spacing, and
        (ii) a second light source in contact with a third location on the anatomical wall spaced from said second location by a distance less than said first spacing, and

    (b) employing said additional measurements to assess a contribution to said PPG curve due to the blood in the tissue of said anatomical wall.

13. A device for determining oxygen saturation $SvO_2$ in the blood of the pulmonary microcirculatory arteries of a subject, the device comprising the components:

    (a) a first light source of a first wavelength in the infrared;
    (b) a second light source of a second wavelength in the infrared, different from said first wavelength;
    (c) means for directing the light of the two wavelengths into the same tissue of the lungs;
    (d) a detector for detecting part of the light for each of said first and second wavelengths that has passed through microcirculatory tissue of the lungs and generating two signals which are functions of intensities of the detected light for each of said first and second wavelengths; and
    (e) processing means for processing said signals to derive PPG curves for pulmonary microcirculatory arteries and for processing said PPG curves for deriving a ratio R between values of a parameter related to relative changes in light transmission for said first and second wavelengths, and determining from said ratio R the oxygen saturation in the blood of the pulmonary microcirculatory arteries;

    wherein said first and second light source are in contact with a first location on the thoracic wall of the subject, and said detector is in contact with a second location on the thoracic wall of the subject wherein said second location is at least 15 millimeters from said first location.

14. The device of claim 13, wherein said light of said first wavelength is the peak of an emission spectrum of a first infrared light source and said infrared light of said second wavelength is a peak of an emission spectrum of a second infrared light source, and wherein said processing means determines said oxygen saturation $SvO_2$ in the blood of the pulmonary microcirculatory arteries from R based on a relationship including the mean value of the extinction coefficient for each of the two emission spectra of the two light sources, for oxygenated blood $\varepsilon_o$ and for deoxygenated blood $\varepsilon_d$.

**15.** The device of claim 14, wherein the relationship between oxygen saturation in the blood of the pulmonary microcirculatory arteries $SvO_2$ and said ratio R which includes the mean value of the extinction coefficient for each of the two wavelength spectra for oxygenated blood $\varepsilon_o$ and for deoxygenated blood $\varepsilon_d$, is

$$SvO_2 = \frac{\varepsilon_{d1} - R\varepsilon_{d2}}{R(\varepsilon_{02} - \varepsilon_{d2}) + (\varepsilon_{d1} - \varepsilon_{01})} .$$

Figure 1a

Figure 1b

EP 2 666 412 A1

Figure 2.

Figure 3

Figure 4

Figure 5

a        b

Figure 6

Figure 7

Europäisches
Patentamt

European
Patent Office

Office européen
des brevets

# EUROPEAN SEARCH REPORT

Application Number

EP 13 16 8402

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/098353 A1 (ACMED TECHNOLOGY INC [CA]; CHENG XUEFENG [CA]) 21 August 2008 (2008-08-21) | 13-15 | INV. A61B5/1455 A61B5/00 G01N33/49 |
| A | * paragraphs [0015], [0028], [0042] - [0045], [0049] * | 1-12 | |
| X | US 2004/082841 A1 (FURNARY ANTHONY P [US] ET AL) 29 April 2004 (2004-04-29) | 13-15 | |
| A | * the whole document * | 1-12 | |
| X,D | US 5 329 922 A (ATLEE III JOHN L [US]) 19 July 1994 (1994-07-19) | 13-15 | |
| A | * the whole document * | 1-12 | |
| X | DE 44 42 260 A1 (MIPM MAMMENDORFER INST FUER PH [DE]) 30 May 1996 (1996-05-30) | 13-15 | |
| A | * the whole document * | 1-12 | |
| A | US 2004/024297 A1 (CHEN BO [US] ET AL) 5 February 2004 (2004-02-05) * the whole document * | 1-15 | |
| A | US 5 193 544 A (JAFFE RICHARD A [US]) 16 March 1993 (1993-03-16) * the whole document * | 1-15 | TECHNICAL FIELDS SEARCHED (IPC) A61B G01N |
| A,D | M. NITZAN; A. BABCHENKO; B. KHANOKH; H, TAITELBAUM: "Measurement of oxygen saturation in venous blood by dynamic near IR spectroscopy", J. BIOMED. OPTICS., vol. 5, 2000, pages 155-162, XP002711580, * the whole document * | 1-15 | |

The present search report has been drawn up for all claims

| Place of search | Date of completion of the search | Examiner |
|---|---|---|
| The Hague | 22 August 2013 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 13 16 8402

This annex lists the patent family members relating to the patent documents cited in the above-mentioned European search report.
The members are as contained in the European Patent Office EDP file on
The European Patent Office is in no way liable for these particulars which are merely given for the purpose of information.

22-08-2013

| Patent document cited in search report | | Publication date | Patent family member(s) | | Publication date |
|---|---|---|---|---|---|
| WO 2008098353 | A1 | 21-08-2008 | AU | 2008215133 A1 | 21-08-2008 |
| | | | CA | 2676271 A1 | 21-08-2008 |
| | | | EP | 2120689 A1 | 25-11-2009 |
| | | | JP | 2010517711 A | 27-05-2010 |
| | | | US | 2008200784 A1 | 21-08-2008 |
| | | | US | 2009326352 A1 | 31-12-2009 |
| | | | WO | 2008098353 A1 | 21-08-2008 |
| US 2004082841 | A1 | 29-04-2004 | AT | 489032 T | 15-12-2010 |
| | | | AU | 2003301517 A1 | 13-05-2004 |
| | | | CA | 2503197 A1 | 06-05-2004 |
| | | | EP | 1558133 A2 | 03-08-2005 |
| | | | US | 2004082841 A1 | 29-04-2004 |
| | | | US | 2006149145 A1 | 06-07-2006 |
| | | | WO | 2004037319 A2 | 06-05-2004 |
| US 5329922 | A | 19-07-1994 | NONE | | |
| DE 4442260 | A1 | 30-05-1996 | DE | 4442260 A1 | 30-05-1996 |
| | | | US | 5817009 A | 06-10-1998 |
| US 2004024297 | A1 | 05-02-2004 | AU | 2003254135 A1 | 16-02-2004 |
| | | | CA | 2494030 A1 | 05-02-2004 |
| | | | EP | 1545298 A2 | 29-06-2005 |
| | | | JP | 4465271 B2 | 19-05-2010 |
| | | | JP | 2005533609 A | 10-11-2005 |
| | | | JP | 2010088928 A | 22-04-2010 |
| | | | US | 2004024297 A1 | 05-02-2004 |
| | | | US | 2006189861 A1 | 24-08-2006 |
| | | | US | 2012108927 A1 | 03-05-2012 |
| | | | WO | 2004010844 A2 | 05-02-2004 |
| US 5193544 | A | 16-03-1993 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060253007 A
- US 20100198027 A
- US 6961600 B2
- US 4773422 A
- US 4167331 A
- US 6505060 B
- US 5329922 A
- US 20080045822 A, Phillips

### Non-patent literature cited in the description

- **SCOTT PRAHL.** *Oregon Medical Laser Center,* 1999
- **M. NITZAN ; H. TAITELBAUM.** *IEEE Instrumentation and Measurement Magazine,* 2008, vol. 11, 9-15
- **KIM ; LIU.** *Phys. Med. Biol.,* 2007, vol. 52, 6295-6322
- **I. YOSHIYA ; Y. SHIMADY ; K. TANAKE.** Spectrophotometric monitoring of arterial oxygen saturation on the fingertip. *Med. Biol. Eng, Comput.,* 1980, vol. 18, 27-32
- **A. BABCHENKO ; E. DAVIDSON ; Y. GINOSAR ; V. KURTZ ; I. FEIB ; D. ADLER ; M. NITZAN.** Photoplethysmographic measurement of changes in total and pulsatile tissue blood volume following sympathetic blockade. *Physiol. Meas.,* 2001, vol. 22, 389-396
- **K. H. SHELLEY.** Photoplethysmography: beyond the calculation of arterial oxygen saturation and heart rate. *Anesth Analg,* 2007, vol. 105, 31-6
- **M.N. NITZAN ; A. BABCHENKO ; B. KHANOKH ; D. LANDAU.** The variability of the photoplethysmographic signal - a potential method for the evaluation of the autonomic nervous system. *Physiol. Meas.,* 1998, vol. 19, 93-102
- Light absorbance in pulse oximetry. **O. WIEBEN.** Design of Pulse Oximeters. Institute of Physics Publishing, 1997, 40-55
- **P. D. MANNHEIMER ; J. R. CASCIANI ; M. E. FEIN ; S. L. NIERLICH.** Wavelength selection for low-saturation pulse oximetry. *IEEE Trans. Biomed. Eng.,* 1997, vol. 44 (3), 148-158
- **M. NITZAN ; A. BABCHENKO ; B. KHANOKH ; H. TAITELBAUM.** The measurement of oxygen saturation in venous blood by dynamic near IR spectroscopy. *J. Biomed. Optics.,* 2000, vol. 5, 155-162
- Calibration. **SCHOWALTER JS.** Design of Pulse Oximeters. Institute of Physics Publishing, 1997, 159-175
- **A. DUNCAN ; J.H. MEEK ; M. CLEMENCE ; C.E. ELWELL ; L. TYSZCZUK ; M. COPE ; D.T. DELPY.** Optical pathlength measurements on adult head, calf and forearm and the head of the newborn infant using phase resolved spectroscopy. *Phys. Med. Biol.,* 1995, vol. 40, 295-304
- **J.L. REUSS ; D. SIKER.** The pulse in reflectance pulse oximetry: modeling and experimental studies. *J Clin Monit Comput.,* 2004, vol. 18, 289-99
- **P. D. MANNHEIMER ; O' NM ; E. KONECNY.** The influence of larger subcutaneous blood vessels on pulse oximetry. *J Clin Monit Comput.,* 2004, vol. 18, 179-88
- **N.B. KARATZAS ; G.J. LEE.** Propagation of blood flow pulse in the normal human pulmonary arterial system. *Circ, Res.,* 1969, vol. 15, 11-21
- **C. HER ; D. HAYS ; D.E. LEES.** Elevated pulmonary artery systolic storage volume associated with redistribution of pulmonary perfusion. *Crit. Care Med,* 1987, vol. 15, 1023-9
- **M. NITZAN ; Y. MAHLER ; S. YAFFE ; R. MARZIANO ; M. BOCHER ; R. CHISHIN.** ECG-gated radionuclide plethysmography - a method for the assessment of pulmonary systolic blood volume increase. *Clin. Phys. Physiol Meas,* 1992, vol. 13, 21-8
- **M. NITZAN ; Y. MAHLER ; D. SCHECHTER ; S. YAFFE ; M. BOCHER ; R. CHISHIN.** A measurement of pulmonary blood volume increase during systole in humans. *Physiol Meas.,* 1994, vol. 15, 489-498
- **M. UGANDER ; E. JENSE ; H. ARHEDEN.** Pulmonary intravascular blood volume changes through the cardiac cycle in healthy volunteers studied by cardiovascular magnetic resonance measurements of arterial and venous flow. *J. Cardiovasc. magnetic resonance,* 2009, vol. 11, 42
- **P.A. KYRIACOU.** Pulse oximetry in the esophagus. *Physiol Meas.,* 2006, vol. 27, 1-35

- **J.P. PHILLIPS ; R.M. LANGFORD ; S.H. CHANG ; P.A. KYRIACOU ; D.P. JONES.** Photoplethysmographic measurements from the esophagus using a new fiber-optic reflectance sensor. *J Biomed Opt.,* 2011, vol. 16 (7), 077005